# EUROPEAN PATENT SPECIFICATION

(11) **EP 4 295 758 B1**
(45) Date of publication and mention of the grant of the patent: **08.10.2025**
(21) Application number: 23757503.0
(22) Date of filing: 10.03.2023
(51) Int. Cl.: A61B 5/02

(54) **METHOD AND APPARATUS FOR ALLEVIATING VENTILATOR WITHDRAWAL BASED ON GAS MIXTURE**
VERFAHREN UND VORRICHTUNG ZUR VERMINDERUNG DES ENTZUGS EINES BEATMUNGSGERÄTES BASIEREND AUF EINEM GASGEMISCH
PROCÉDÉ ET APPAREIL POUR ATTÉNUER LE SEVRAGE D'UN VENTILATEUR MÉCANIQUE SUR LA BASE D'UN MÉLANGE GAZEUX

(30) Priority: 05.05.2022 CN 202210480182
(43) Date of publication of application: 27.12.2023
(73) Proprietor: Guangzhou Landswick Medical Technologies Ltd., Guangzhou, Guangdong 510000 (CN)
(72) Inventor: DONG, Hui, Guangzhou, Guangdong 510000 (CN); ZHAO, Longchao, Guangzhou, Guangdong 510000 (CN); SUN, Caixin, Guangzhou, Guangdong 510000 (CN)
(74) Representative: Ipside
(86) International application number: PCT/CN2023/080731
(87) International publication number: WO 2023/213138

(56) References cited:
- WO-A1-2023/213154
- CN-A- 103 893 866
- CN-A- 106 659 436
- CN-A- 109 718 443
- CN-A- 111 991 663
- CN-A- 112 675 393
- CN-A- 114 038 563
- CN-A- 114 887 184
- US-A1- 2016 121 064
- US-A1- 2021 170 136

## Description

### Technical Field

The present invention relates to the field of ventilator technology, and more particular to a device for solving patient ventilator off-line problems on patients based on mixed gases.

### Background of the Invention

At present, when patients use a ventilator (usually oxygen O2 and air or mixed gas) to support their own breathing for a long time, their respiratory system function will degrade, they will lose their ability to breathe spontaneously, and "addiction" will appear. When the patient needs to be taken off-line, it is necessary to gradually restore the patient's own respiratory function with the help of certain functions of the ventilator. The recovery period is long, and the curative effect is unstable. Even off-line, there are often repetitions, that is, to use the ventilator again to support self-breathing. Increased patient distress during recovery of breathing;

Therefore, the present invention provides a device for solving the offline of the patient's ventilator based on mixed gas. By mixing a certain concentration of carbon dioxide gas and oxygen, the patient's respiratory nerve center can be stimulated, thereby ensuring that the patient is stimulated to carry out active breathing. , improve the stability of the treatment, ensure the effect and efficiency of the patient's respiratory function recovery, and reduce the pain of the patient when off-line.

Patent document US 2016/121064 A1 discloses a system for estimating the respiratory drive in mechanically ventilated patients by analyzing changes in respiratory feedback and volume/pressure settings, using a physiological model to apportion the drive into chemical (related to chemoreceptor response) and muscular (related to respiratory muscle action) components.

Patent document WO 2023/213154 A1 discloses a method and system aimed at mitigating the effects of hyperventilation-induced respiratory alkalosis during mechanical ventilation. By closely monitoring the patient's carbon dioxide partial pressure (PCO2), the system can detect early signs of respiratory alkalosis. Upon detection, the system actively adjusts the ratio of oxygen to carbon dioxide in the breathing circuit, effectively normalizing the patient's blood gases and acid-base balance.

### Summary of the Invention

The invention is set out in the appended claim. The invention provides a device for solving the off-line of a patient's ventilator based on mixed gas, which is used to stimulate the patient's respiratory nerve center by mixing a certain concentration of carbon dioxide gas with oxygen, thereby ensuring that the patient is stimulated to carry out active breathing. It improves the stability of the treatment, ensures the effect and efficiency of the patient's respiratory function recovery, and reduces the pain of the patient when offline.

It is also disclosed an unclaimed method for solving the off-line of the patient's ventilator based on the mixed gas, comprising:
Step (1): Mix carbon dioxide and oxygen based on the initial mixing concentration and deliver the mixture to the patient based on the ventilator device;
Step (2): Monitor the patient's partial pressure of carbon dioxide in real time based on the delivery result, and change the initial mixed concentration of carbon dioxide and oxygen in a gradient based on the partial pressure of carbon dioxide;
Step (3): Obtain the patient's respiratory muscle function data based on the gradient change result, and evaluate the respiratory muscle function data, and complete the offline of the patient's ventilator based on the evaluation result.

Preferably, a method for solving the off-line of a patient's ventilator based on mixed gas, in step (1), mixing carbon dioxide and oxygen based on the initial mixed concentration, comprising:
obtaining current working parameters of the ventilator device, and determining the target oxygen supply mode of the ventilator device based on the current working parameters, wherein the target oxygen supply mode is a standard oxygen supply mode or an offline oxygen supply mode;
when the target oxygen supply mode is a standard oxygen supply mode, switching the target oxygen supply mode of the ventilator device based on a preset switching valve; when the target oxygen supply mode is an offline oxygen supply mode, obtaining the control range of the breathing apparatus for the carbon dioxide concentration, and determining the initial mixed concentration of carbon dioxide based on the control range; and
based on the initial mixing concentration, the carbon dioxide and oxygen are respectively input into the target container through the airway tube for mixing to obtain a mixed gas of carbon dioxide and oxygen.

Preferably, a method for solving the off-line of the patient's ventilator based on the mixed gas, based on the initial mixed concentration, respectively mixing the carbon dioxide and oxygen into the target container through the airway tube, comprising:
sampling the carbon dioxide based on a preset sampling device, and detecting the concentration of the sampled carbon dioxide to obtain the detection concentration of the carbon dioxide;
comparing the detected concentration with the initial mixed concentration;
wherein if there is a difference between the detected concentration and the initial mixed concentration, it is determined that there is a defect in the mixing of carbon dioxide and oxygen, and an alarm operation is performed, and the operation of inputting the mixed gas to the patient is terminated simultaneously;
otherwise, it is determined that the mixing of carbon dioxide and oxygen is qualified, and the process of mixing carbon dioxide and oxygen is detected in real time until the patient is off the ventilator.

Preferably, a method for solving a patient ventilator off-line based on a mixed gas, in step (1), delivering the mixed gas to the patient based on the ventilator device, comprising:
acquiring the patient's sign parameters, and determine the oxygen supply pressure and oxygen supply rate to the patient based on the sign parameters;
adjusting the operating parameters of the ventilator device based on the oxygen supply pressure and oxygen supply rate, and detecting the airtightness of the inspiratory tube of the ventilator device based on the adjustment result;
delivering a mixture of carbon dioxide and oxygen to the patient based on the inspiratory tube if the airtight seal is intact; and
otherwise, replacing the inspiratory tube, and delivering the mixed gas to the patient based on the result of the replacement.

Preferably, a method for solving the off-line of a patient ventilator based on a mixed gas, based on the inspiratory tube The mixed gas of carbon dioxide and oxygen is delivered to the patient, comprising:
obtaining the time information of the patient receiving the mixed gas of carbon dioxide and oxygen and the gas volume of the mixed gas, and obtain the identity information of the patient; and
constructing the patient treatment electronic data sheet based on the time information, gas volume and identity information, and typesetting the patient treatment electronic data table to obtain the target recording areas of the time information, gas volume and identity information respectively;

The time information, gas volume and identity information are recorded based on the target recording area, and the gas volume of the mixed gas received by the patient in the patient treatment electronic data sheet is updated synchronously.

Preferably, a method for solving the off-line of the patient's ventilator based on the mixed gas, in step (2), the partial pressure of carbon dioxide of the patient is monitored in real time based on the delivery result, and the initial mixed concentration of carbon dioxide and oxygen is gradually changed based on the partial pressure of carbon dioxide, comprising:
Based on the ventilator device, the mixed gas of the initial mixed concentration is continuously input to the patient for a preset period of time, and the partial pressure of carbon dioxide of the patient is monitored in real time, wherein the partial pressure of carbon dioxide comprises arterial partial pressure of carbon dioxide and end-tidal partial pressure of carbon dioxide;
determining the target value of the partial pressure of carbon dioxide of the patient at different time points based on the monitoring results, and obtaining a numerical sequence of the partial pressure of carbon dioxide of the patient within a preset time period;
matching the target chart from the preset chart template library based on the numerical sequence, and determining the horizontal and vertical coordinate legends of the two-dimensional coordinates of the target chart based on the attribute information of the preset time period and the target value of the numerical sequence, Among them, the legend on the abscissa is time, and the legend on the ordinate is the target value of the numerical sequence;
determining the configuration parameters of the target chart, and performing format conversion on the numerical sequence of the partial pressure of carbon dioxide based on the configuration parameters to obtain the partial pressure of carbon dioxide visual graph;
determining the maximum value and the minimum value of the partial pressure of carbon dioxide of the patient in the preset time period based on the partial pressure of carbon dioxide visual graph, and determine the carbon dioxide concentration of the patient based on the maximum value and the minimum value of carbon dioxide Pressure fluctuation range;
comparing the stated fluctuation range with the standard allowable range;
wherein if the fluctuation range is not within the allowable range of the standard, it is determined that the patient's inhalation of the mixed gas is abnormal;
otherwise, it is determined that there is no abnormality in the patient's inhalation of the mixed gas, and at the same time, the pH value in the patient's body is obtained;
determining the difference between the pH value and the standard pH value, and when the difference is within the preset allowable fluctuation range, it is determined that the patient has adapted to the mixed gas of the initial mixed concentration, and the ventilator Device feedback judgment result.

Based on the received feedback determination result, control the ventilator device to change the initial mixed concentration of carbon dioxide and oxygen by gradient, and continuously monitor the partial pressure of carbon dioxide of the patient until the initial mixed concentration reaches the gradient change The maximum allowed value, wherein the allowed range of the gradient change is 1%-6%.

Preferably, a method for solving the off-line of the patient ventilator based on the mixed gas, so as to judge that there are abnormalities in the inhalation of the gas mixture by the patients, comprising:
obtaining the comparison result between the fluctuation range and the standard allowable range, and when it is determined that the patient's inhalation of the mixed gas is abnormal, generate a target control instruction;
based on the target control instruction, controlling the ventilator device to stop delivering the mixed gas to the patient, and collecting a preset amount of the mixed gas, and at the same time acquiring the current sign data of the patient, wherein the sign data at least one;
detecting the mixed gas, respectively determining the ratio of carbon dioxide and oxygen in the mixed gas example, and verifying the ratio based on the preset ratio based on the ratio preset;
wherein if it fails to pass the verification, it is determined that the ventilator device has a defect in the mixing process of the carbon dioxide and oxygen, and a first alarm is carried out; and
if the verification is passed, then the current sign data of the patient is analyzed to determine whether there is any abnormality in the current characteristic data of the patient, and if there is any abnormality, the second alarm is carried out;
recording first alarm and the second alarm respectively to obtain the abnormal situation report sheet of the patient in the off-line process of the ventilator.

Preferably, a method for solving the off-line of the patient's ventilator based on the mixed gas, in step (3), the patient's respiratory muscle function data is obtained based on the gradient change result, and the respiratory muscle function data is evaluated, and the patient's respiratory muscle function data is evaluated based on the evaluation result. Ventilator off-line, comprising:
obtaining the respiratory waveform data of the patient after gradiently changing the initial mixed concentration of the mixed gas based on the preset sensor, and fitting the respiratory waveform data to obtain the respiratory wave curve of the patient;
obtaining a reference respiratory wave curve, and superimposing the reference respiratory wave curve with the patient's respiratory wave curve, wherein the reference respiratory wave curve is obtained from training based on historical training data;
determining a difference area between the respiratory wave curve and the reference respiratory wave curve based on the superposition result
domain, and extract the waveform features of the respiratory wave curve in the difference area;
determining curve parameters of the patient's respiratory wave curve in the difference area based on the waveform features, and treating the curve parameters to obtain the breathing data of the patient in the difference area;
extracting the attribute information of the respiratory data, and matching the target data cleaning rules from the preset data cleaning rule base based on the attribute information;
cleaning the breathing data based on the target data cleaning rules to obtain the respiratory muscle function data of the patient;
constructing a spontaneous breathing capacity assessment model, and inputting the respiratory muscle function data into the spontaneous breathing capacity assessment model for processing, determining the current respiratory rate of the patient, and simultaneously determining the respiratory intensity of the patient based on the current respiratory rate;
Respectively determining the weight of the respiratory rate and respiratory intensity in the evaluation process of spontaneous breathing ability value, and obtaining the comprehensive evaluation value of the patient's current spontaneous breathing ability based on the weight value;
dispalying comprehensive evaluation value of the patient's current spontaneous breathing ability on the ventilator device, and at the same time, transmitting the comprehensive evaluation value of the patient's current spontaneous breathing ability to the doctor's smart terminal to complete the evaluation of the patient's spontaneous breathing ability.

Preferably, a method for solving the off-line of the patient's ventilator based on the mixed gas, wherein based on the weight value, obtaining the comprehensive evaluation value of the patient's current spontaneous breathing ability, comprising:
obtaining the comprehensive evaluation value of the patient's current spontaneous breathing ability, and comparing the comprehensive evaluation value of the patient's current spontaneous breathing ability with a preset offline threshold;
wherein if the comprehensive evaluation value of the patient's current spontaneous breathing ability is less than the preset offline threshold, it is determined that the patient does not meet the offline requirements, and at the same time, determine that the patient's current comprehensive evaluation value of spontaneous breathing ability is different from the preset offline threshold a threshold difference, and based on the difference, estimate the duration of use of the ventilator device by the patient;
feeding back the estimated result to the patient's smart terminal;
otherwise it is determined that the patient meets the off-line requirement, and the off-line operation on the patient is completed.

Preferably, a device for solving the off-line of the patient's ventilator based on the mixed gas comprising:
a gas mixing module configure to mix carbon dioxide and oxygen based on the initial mixing concentration and deliver the mixed gas to the patient based on the ventilator device;
a concentration changing module configured to monitor the patient's partial pressure of carbon dioxide in real time based on the delivery result, and to change the initial mixed concentration of carbon dioxide and oxygen in a gradient based on the partial pressure of carbon dioxide; and
an evaluation module configured to obtain the patient's respiratory muscle function data based on the gradient change result, evaluate the respiratory muscle function data, and complete the patient's ventilator off-line based on the evaluation result.

### Brief Description of the Drawings

In the attached picture:
Fig. 1 is a flowchart of an unclaimed method for solving a patient ventilator off-line based on mixed carbon dioxide gas;
Fig. 2 is a schematic diagram of an unclaimed method for solving a patient ventilator off-line based on mixed carbon dioxide gas;
Fig. 3 is a structural diagram of a device for solving a patient's ventilator off-line based on mixed carbon dioxide gas in an embodiment of the present invention.

### Detailed Description of Embodiments

### Embodiment 1:

### comprising:s

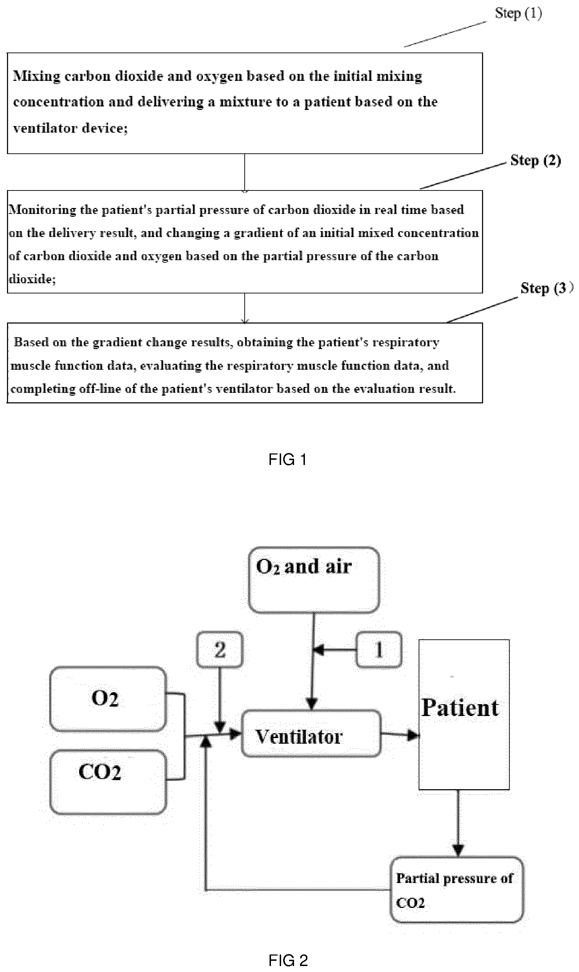

As shown in Fig. 1, the embodiment 1 provides a method for solving ventilator offline problems on patients based on mixed gases, comprising:
Step (1): mixing carbon dioxide and oxygen based on the initial mixing concentration and delivering the mixture to the patient based on the ventilator device;
Step (2): monitoring the patient's partial pressure of carbon dioxide in real time based on the delivery result, and changing the initial mixed concentration of carbon dioxide and oxygen in a gradient based on the partial pressure of carbon dioxide; and
Step (3): obtaining the patient's respiratory muscle function data based on the gradient change result, and evaluating the respiratory muscle function data, and complete the off-line of the patient's ventilator based on the evaluation result.

In this embodiment, the initial mixed concentration refers to the mixing of the minimum control concentration of carbon dioxide and oxygen according to the ventilator device, in order to allow the patient to adapt from the lowest carbon dioxide concentration, thereby get off the ventilator.

In this embodiment, the partial pressure of carbon dioxide refers to the partial pressure of the patient's end-tidal carbon dioxide (EtCO2) and arterial carbon dioxide (PaCO2); wherein the partial pressure of the patient's end-tidal carbon dioxide (EtCO2) is configured to indicate ventilation condition in lungs of the patients, and arterial carbon dioxide (PaCO2) is configured to indicate an amount of carbon dioxide in the patient's arterial blood.

In this embodiment, the gradient change refers to increasing the concentration of carbon dioxide, and the concentration increasing range is 1%-6%.

In this embodiment, the respiratory muscle function data refers to the patient's ability to breathe spontaneously after receiving the mixed gas of carbon dioxide and oxygen at different concentrations, including the breathing rate and so on.

In this embodiment, completing the weaning of the ventilator for the patient based on the evaluation results means that the weaning of the ventilator can be performed when the patient's respiratory muscles can reach a normal standard.

The beneficial effect of the above technical solution is: by mixing a certain concentration of carbon dioxide gas and oxygen, the patient's respiratory nerve center can be stimulated, so as to ensure the stimulation of the patient's active breathing, improve the stability of the treatment, and ensure the recovery of the patient's respiratory function. The effect and efficiency of the machine can reduce the pain of patients when they are offline.

### Embodiment 2:

On the basis of the above-mentioned embodiment 1, this embodiment provides a method for solving the off-line of the patient's ventilator based on mixed gas, as shown in Figure 2, in step (1), carbon dioxide and oxygen are mixed based on the initial mixed concentration, comprising:
obtaining the current working parameters of the ventilator device, and determining the target oxygen supply mode of the ventilator device based on the current working parameters, wherein the target oxygen supply mode is a standard oxygen supply mode or an offline oxygen supply mode;
when the target oxygen supply mode is a standard oxygen supply mode, switch the target oxygen supply mode of the ventilator device based on a preset switching valve;
when the target oxygen supply mode is an offline oxygen supply mode, obtain the control range of the breathing apparatus for the carbon dioxide concentration, and determine the initial mixed concentration of carbon dioxide based on the control range; and
based on the initial mixing concentration, respectively inputting the carbon dioxide and oxygen into the target container through the airway tube for mixing to obtain a mixed gas of carbon dioxide and oxygen.

In this embodiment, the current working parameter may be the type of the current gas supply source of the ventilator device and the concentration or ratio of the gas supply to different gas supply sources.

In this embodiment, the target oxygen supply mode comprise two modes, namely a standard oxygen supply mode and an offline oxygen supply mode, wherein the standard oxygen supply mode is to mix oxygen with air,. and the offline oxygen supply mode is to mix oxygen with carbon dioxide.

In this embodiment, the preset switching valve is set in advance in the ventilator device and is used to switch the oxygen supply mode.In this embodiment, the control range is 1%-6%.

In this embodiment, the target container is pre-set in the ventilator device to fully mix the oxygen and carbon dioxide.

The beneficial effect of the above technical solution is: by determining the oxygen supply mode of the ventilator device, and when the oxygen supply mode of the ventilator device is the standard oxygen supply mode, the oxygen supply mode of the ventilator device is switched when it needs to be switched, and at the same time When switching to the offline oxygen supply mode, the carbon dioxide and oxygen are mixed, which facilitates stable treatment of the patient and ensures the recovery of the patient's respiratory function.

### Embodiment 3:

On the basis of the above-mentioned embodiment 2, this embodiment provides a method based on the mixed gas to solve the off-line of the patient's ventilator. Based on the initial mixed concentration, respectively mixing the carbon dioxide and oxygen into the target container through the airway tube, comprises:
sampling the carbon dioxide based on a preset sampling device, and detecting the concentration of the sampled carbon dioxide to obtain the detection concentration of the carbon dioxide;
comparing the detected concentration with the initial mixed concentration;
if there is a difference between the detected concentration and the initial mixed concentration, it is determined that there is a defect in the mixing of carbon dioxide and oxygen, and an alarm operation is performed, and the operation of inputting the mixed gas to the patient is terminated simultaneously;
otherwise, it is determined that the mixing of carbon dioxide and oxygen is qualified, and the process of mixing carbon dioxide and oxygen is detected in real time until the patient is off the ventilator.

In this embodiment, the preset sampling device is set in advance and is used for random sampling of carbon dioxide, the purpose of which is to detect whether the concentration of carbon dioxide is consistent with the initial mixed concentration.

The beneficial effects of the above technical solution are as follows.: During the mixing process of carbon dioxide and oxygen, random collection of carbon dioxide and detection of the current concentration ensures strict control of the concentration of carbon dioxide, and improves the offline security of ventilators for patients by mixing carbon dioxide gas.

### Embodiment 4:

On the basis of the above-mentioned embodiment 1, this embodiment provides a method based on the mixed gas to solve the patient's ventilator offline. In step (1), the mixed gas is delivered to the patient based on the ventilator device, comprising:
acquiring the patient's sign parameters, and determine the oxygen supply pressure and oxygen supply rate to the patient based on the sign parameters;
adjusting the operating parameters of the ventilator device based on the oxygen supply pressure and oxygen supply rate, and detecting the airtightness of the inspiratory tube of the ventilator device based on the adjustment result;
wherein if the airtightness is intact, the mixed gas of carbon dioxide and oxygen is delivered to the patient;
otherwise, the inspiratory tube is replaced, and the mixed gas is delivered to the patient based on the result of the replacement.

In this embodiment, the sign parameters refer to the patient's pulse, heart rate, and blood pressure.

In this embodiment, the operating parameters refer to parameters such as working power and working current of the ventilator device. In this embodiment, the airtightness is used to characterize whether the suction pipe leaks.

The beneficial effect of the above technical solution is: by analyzing the patient's sign parameters, the oxygen supply pressure and the oxygen supply rate of the ventilator device can be adjusted according to the patient's signs, so as to ensure the stable and effective delivery of the mixed gas to the patient. The treatment effect of the patient is guaranteed, and at the same time, it is convenient for the patient to quickly recover the spontaneous breathing function, and the efficiency of the off-line ventilator is improved.

### Embodiment 5:

On the basis of the above-mentioned embodiment 4, this embodiment provides a method for solving the off-line of the patient's ventilator based on the mixed gas, and the mixed gas of carbon dioxide and oxygen is delivered to the patient based on the inspiratory tube, comprising:
obtaining the time information of the patient receiving the mixed gas of carbon dioxide and oxygen and the gas volume of the mixed gas, and obtain the identity information of the patient;
constructing the patient treatment electronic data sheet based on the time information, gas volume and identity information, and typesetting the patient treatment electronic data table to obtain the target recording areas of the time information, gas volume and identity information respectively; and
recording time information, gas volume and identity information based on the target recording area, and updating the gas volume of the mixed gas received by the patient in the patient treatment electronic data sheet in real time.

In this embodiment, the identity information comprises the patient's name, age and ID number.

In this embodiment, the patient treatment electronic data sheet is used to record various parameters of the patient when receiving the mixed gas treatment and the patient's identity information, so as to facilitate an intuitive understanding of the patient's acceptance of the mixed carbon dioxide gas.

In this embodiment, typesetting refers to setting the table style and data record format in the patient treatment electronic data sheet accordingly.

In this embodiment, the target recording area refers to the cells of the patient treatment electronic data sheet for recording time information, gas volume and identity information respectively.

The beneficial effect of the above technical solution is: by recording the time, gas volume and identity information of the patient receiving the mixed gas, it is convenient to check the current situation of the patient receiving the mixed gas intuitively, and at the same time, it is convenient to timely accept the patient according to the electronic data sheet. The concentration of the mixed gas is adjusted to improve the rigor and safety for patients to solve the off-line of the ventilator by mixing carbon dioxide gas.

### Embodiment 6:

On the basis of the above-mentioned embodiment 1, this embodiment provides a method based on mixed gas to solve the off-line of the patient's ventilator. In step (2), monitoring the partial pressure of carbon dioxide of the patient in real time based on the delivery result, and based on the partial pressure of carbon dioxide Gradient changes in the initial mixed concentration of carbon dioxide and oxygen, comprising:
based on the ventilator device, continuously inputting the mixed gas of the initial mixed concentration to the patient for a preset period of time, and the partial pressure of carbon dioxide of the patient is monitored in real time, wherein the partial pressure of carbon dioxide comprises arterial partial pressure of carbon dioxide and end-tidal partial pressure of carbon dioxide;
determining the target value of the partial pressure of carbon dioxide of the patient at different time points based on the monitoring results, and obtaining a numerical sequence of the partial pressure of carbon dioxide of the patient within a preset time period;
matching the target chart from the preset chart template library based on the numerical sequence, and determining the horizontal and vertical coordinate legends of the two-dimensional coordinates of the target chart based on the attribute information of the preset time period and the target value of the numerical sequence, wherein the legend on the abscissa is time, and the legend on the ordinate is the target value of the numerical sequence;
determining the configuration parameters of the target chart, and performing format conversion on the numerical sequence of the partial pressure of carbon dioxide based on the configuration parameters to obtain the partial pressure of carbon dioxide visual graph; and
determining the maximum value and the minimum value of the partial pressure of carbon dioxide of the patient in the preset time period based on the partial pressure of carbon dioxide visual graph, and determine the maximum value and the minimum value based on the maximum value and the minimum value.

The fluctuation range of the patient's partial pressure of carbon dioxide;
Comparing the stated fluctuation range with the standard allowable range;
If the fluctuation range is not within the allowable range of the standard, it is determined that the patient's inhalation of the mixed gas is abnormal;
Otherwise, it is determined that there is no abnormality in the patient's inhalation of the mixed gas, and at the same time, the pH value in the patient's body is obtained;
Determine the difference between the pH value and the standard pH value, and when the difference is within the preset allowable fluctuation range, it is determined that the patient has adapted to the mixed gas of the initial mixed concentration, and the ventilator Device feedback judgment result;
Based on the received feedback determination result, control the ventilator device to change the initial mixed concentration of carbon dioxide and oxygen by gradient, and continuously monitor the partial pressure of carbon dioxide of the patient until the initial mixed concentration reaches a maximum allowed value of the gradient change, wherein the allowed range of the gradient change is 1%-6%.

In this embodiment, the principle of monitoring the partial pressure of carbon dioxide of the patient in real time and gradiently changing the initial mixed concentration of carbon dioxide and oxygen based on the partial pressure of carbon dioxide is: carbon dioxide maintains pH balance through the reaction of CO2+H2O→H+HCO3, so changes in carbon dioxide will cause changes in pH, all cell functions in the brain and body are generally affected by pH, human life only allows small changes in pH, because the CO2 and H ions produced by metabolism is in a state of rapid equilibrium (CO2/H+ state) and can be cleared by pulmonary ventilation, the dynamic control of respiration by CO2 provides the main homeostatic mechanism for acute regulation of acid-base state.

The mechanism of CO2/H+ homeostasis has implications for the regulation and control of respiration. Low-level hypercapnia can cause irritation of the respiratory system and increased respiratory rate, mainly due to the direct effect of increased CO2/H+ on the carotid body and very few brainstem structures, comprising the posterior trapezium of chemoreceptors Nuclei (RTN) and a subset of serotonergic neurons play a large role.

The CO2/H+ homeostasis mechanism is regulated by the CO2+H2O→H+HCO3 - reaction by inhaling and exhaling CO2. High gain of hypercapnic carbon dioxide (CO2) ventilation chemoreflex caused induces respiratory stimulation, with elevated PaCO2.

The CO2/H+ homeostasis mechanism proceeds through a complex mechanism. At rest, respiratory chemoreflexes initiated at peripheral and central sites mediate rapid stabilization of arterial PaCO2 and pH. Specific brainstem neurons (eg, posterior trapezoidal nucleus, RTN; serotonergic) are activated by PaCO2 and stimulate respiration. RTN neurons detect CO2 through endogenous proton receptors (TASK-2, GPR4), synaptic inputs from peripheral chemoreceptors, and signals from astrocytes. Respiratory chemoreflexes depend on arousal, and stimulation of chemoreceptors leads to arousal. When the two are abnormal, these interactions can lead to sleep-disordered breathing. During exercise, despite increased metabolic activity, central commands and reflexes from the exercising muscles produce the required respiratory stimulation to maintain arterial PaCO2 and pH.

In this embodiment, the purpose of changing the mixed gas concentration gradient is: After the patient inhales the breathing gas containing a certain proportion of carbon dioxide gas, it is absorbed by the human body, so that the pH in the patient's body decreases and the arterial CO2 partial pressure increases. Central chemoreceptor serotonergic neurons (serotonergicneurons) located in the ventral medulla and posterior trapezoidal nucleus sense the decrease in central nervous system pH caused by elevated arterial carbon dioxide. The respiratory centers within the medulla and pons then generate a unified neural signal that is sent to the main muscles of respiration, producing respiration. In this way, the active breathing can be stimulated by inhaling a certain amount of CO2, and the patient's spontaneous breathing function can be quickly and effectively restored.

In this embodiment, the preset time period is set in advance, such as one day, two days, etc., and can be adjusted according to medical conditions.

In this embodiment, the end-tidal partial pressure of carbon dioxide is used to reflect pulmonary ventilation and pulmonary blood flow.

In this embodiment, the target value refers to the specific number of partial pressure of carbon dioxide of the patient at different time points. The value is convenient for an effective understanding of the patient's breathing condition.

In this embodiment, the numerical sequence refers to the sequence obtained by sequentially arranging the target values of the partial pressure of carbon dioxide according to time sequence.

In this embodiment, the preset chart template library is set in advance, and different types of chart templates are stored inside.

In this embodiment, the target chart refers to a chart suitable for displaying the patient's partial pressure of carbon dioxide.

In this embodiment, the attribute information refers to a data type of a preset time period, that is, "time".

In this embodiment, the configuration parameters refer to the composition of the target image and the format requirements of the data to be displayed.

In this embodiment, the visible graph of the partial pressure of carbon dioxide refers to the chart information obtained by displaying the partial pressure of carbon dioxide using a target image.

In this embodiment, the standard allowable range is set in advance and is used to measure whether the patient is breathing normally.

In this embodiment, the preset allowable fluctuation range is set in advance and is used to measure the pH in the patient's body. In the case of changing values, the range is generally small.

In this embodiment, gradient change refers to sequentially increasing the concentration of carbon dioxide from 1% to 6%.

The beneficial effect of the above-mentioned technical solution is: by continuing to perform image visualization on the patient's partial pressure of carbon dioxide

It is easy to accurately analyze the fluctuation range of the patient's partial pressure of carbon dioxide, so as to ensure that the patient's partial pressure of carbon dioxide remains within the normal range. Secondly, judge the pH value in the patient's body, and when the pH change is within the set range At the same time, the gradient change of the patient's carbon dioxide is completed, which ensures the safety of the patient when the carbon dioxide concentration is changed, and at the same time facilitates the patient to quickly and effectively achieve off-line, reducing the pain during off-line.

### Embodiment 7:

On the basis of the above-mentioned embodiment 6, this embodiment provides a method for solving the patient's ventilator offline based on the mixed gas, and it is determined that the patient's inhalation of the mixed gas is abnormal, comprising:
obtaining the comparison result between the fluctuation range and the standard allowable range, and when it is determined that the patient's inhalation of the mixed gas is abnormal, generate a target control instruction;
based on the target control instruction, controlling the ventilator device to stop delivering the mixed gas to the patient, and collecting a preset amount of the mixed gas, and at the same time acquiring the current sign data of the patient, wherein an amount of the sign data is at least one;
detecting the mixed gas, respectively determining the ratio of carbon dioxide and oxygen in the mixed gas example, and verifying the ratio based on the preset ratio;
wherein if it fails to pass the verification, it is determined that the ventilator device has a defect in the mixing process of the carbon dioxide and oxygen, and a first alarm is carried out; and
if the verification is passed, then the current sign data of the patient is analyzed to determine whether there is any abnormality in the current characteristic data of the patient, and if there is any abnormality, the second alarm is carried out;
respectively recording the first alarm and the second alarm to obtain the abnormal situation report sheet of the patient in the off-line process of the ventilator.

In this embodiment, the target control instruction is generated by the ventilator device itself, and is used to control the corresponding device to stop delivering the mixed gas to the patient.

In this embodiment, the preset amount is set in advance and is used for analyzing the ratio of carbon dioxide and oxygen in the mixed gas.

In this embodiment, the sign data refers to the patient's current respiratory rate, pulse, blood pressure, and the like.

In this embodiment, the preset ratio is set in advance and is used to characterize the standard ratio of carbon dioxide and oxygen in the mixed gas.

In this embodiment, the first alarm refers to the alarm performed when there is an abnormality in the mixed gas, which may be an audible alarm.

In this embodiment, the second alarm refers to an alarm performed when there is an abnormality in the patient's sign data, which may be a light alarm.

In this embodiment, the abnormal situation report sheet refers to recording the abnormal situation existing in the process of resolving the ventilator offline, so that patients and doctors can check the abnormal cause in time.

The beneficial effect of the above technical solution is: through the analysis of the ratio of carbon dioxide and oxygen in the mixed gas and the patient's sign data respectively, it is ensured that the cause of the abnormal partial pressure of carbon dioxide is found, thereby ensuring that the patient is in the process of solving the off-line of the ventilator and providing a reliable safety guarantee for patients.

### Embodiment 8:

On the basis of the above-mentioned embodiment 1, this embodiment provides device for solving ventilator off-line problems on patients based on mixed gases in step (3), obtaining the patient's respiratory muscle function data based on the gradient change result, evaluating the respiratory muscle function data, and weaning the patient's ventilator off based on the evaluation result, comprising:
obtaining the respiratory waveform data of the patient after gradiently changing the initial mixed concentration of the mixed gas based on the preset sensor, and fitting the respiratory waveform data to obtain the respiratory wave curve of the patient;
obtaining a reference respiratory wave curve, and superimposing the reference respiratory wave curve with the patient's respiratory wave curve, wherein the reference respiratory wave curve is obtained from training based on historical training data;
determining a difference area between the respiratory wave curve and the reference respiratory wave curve based on the superposition result, and extracting the waveform features of the respiratory wave curve in the difference area;
determining the curve parameters of the patient's respiratory wave curve in the difference area based on the waveform characteristics, and process the curve parameters to obtain the patient's breathing data in the difference area;
extracting the attribute information of the respiratory data, and match the target data cleaning rules from the preset data cleaning rule base based on the attribute information;
cleaning the breathing data based on the target data cleaning rules to obtain the respiratory muscle function data of the patient;
constructing a spontaneous breathing capacity assessment model, and inputting the respiratory muscle function data into the spontaneous breathing capacity assessment model for processing, determining the current respiratory rate of the patient, and simultaneously determining the respiratory intensity of the patient based on the current respiratory rate;
respectively determining the weight of the respiratory rate and respiratory intensity in the evaluation process of spontaneous breathing ability value, and obtaining the comprehensive evaluation value of the patient's current spontaneous breathing ability based on the weight value; and
displaying comprehensive evaluation value of the patient's current spontaneous breathing ability on the ventilator device, and at the same time, transmitting the comprehensive evaluation value of the patient's current spontaneous breathing ability to the doctor's smart terminal to complete the evaluation of the patient's spontaneous breathing ability.

In this embodiment, the preset sensor is set in advance and is used to detect the breathing condition of the patient.

In this embodiment, the reference respiratory wave curve refers to the patient's respiratory wave curve should be, the presented state is obtained by analyzing and training multiple groups of patients' breathing data, and is an average value.

In this embodiment, the difference area refers to the area where there are different curve segments between the patient's respiratory wave curve and the reference respiratory wave curve.

In this embodiment, the waveform feature refers to the curve shape, fluctuation amplitude, etc. of the respiratory wave curve.

In this embodiment, the curve parameter refers to the specific value of the respiratory wave curve in the difference area, comprising the amplitude and period of the respiratory wave curve in this area.

In this embodiment, the attribute information refers to the data type in the respiratory data.

In this embodiment, the preset data cleaning rule library is set in advance, and cleaning rules corresponding to different types of data are stored inside.

In this embodiment, the target data cleaning rule refers to a data cleaning rule suitable for cleaning respiratory data, which is one or more combinations in the preset data cleaning rule library.

**In** this embodiment, the comprehensive evaluation value of the patient's current spontaneous breathing ability is a parameter used to characterize the strength of the patient's spontaneous breathing ability.

**In** this embodiment, obtaining the comprehensive evaluation of the patient's current spontaneous breathing ability based on the weight values, comprising:
obtaining the current respiratory rate of the patient, and calculate the respiratory effort index of the patient based on the respiratory rate, and calculating the comprehensive evaluation value of the patient's current spontaneous breathing capacity based on the respiratory effort index, and the specific steps comprise:
obtaining the lung compliance value and respiratory rate of the patient, and performing dimensionless processing on the lung compliance value and respiratory rate to obtain lung compliance value parameters and respiratory rate parameters;
calculating the respiratory effort index of the patient according to the following formula: 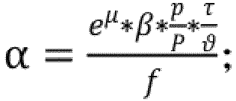
wherein, α represents the respiratory effort index of the patient, and the value range is (1, 30); µ represents the error factor, and the value range is (0.02, 0.05); β represents the lung compliance value parameter of the patient ; *p*ᵣₑₚᵣₑₛₑₙₜₛ the maximum inspiratory pressure of the patient; P represents the airway closure pressure of the patient; τ represents arterial partial pressure of oxygen of the patient; *ϑ* represents the partial pressure of alveolar oxygen of the patient; f represents the respiratory rate parameter of the patient; calculating the comprehensive evaluation value of the patient's current spontaneous breathing capacity according to the following formula: 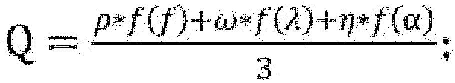
wherein Q represents the comprehensive evaluation value of the patient's current spontaneous breathing ability; *ρ* represents the weight value of the patient's respiratory rate; ***ω*** represents the weight value of the patient's respiratory intensity; *η* represents the weight value of the patient's respiratory effort index; *f*(*f*) presents the patient's respiratory rate evaluation function; *f* (*λ* ) represents the patient's respiratory intensity evaluation function; *f*(*α*) represents the patient's respiratory effort index evaluation function; and *λ* represents the patient's respiratory intensity value;
comparing the comprehensive evaluation value with the preset evaluation value;
wherein if the comprehensive evaluation value is greater than or equal to the preset evaluation value, it is determined that the patient meets the offline condition, processing offline on the patient; otherwise, it is determined that the patient does not meet offline conditions.

The respiratory effort index is a parameter used to reflect the ventilation and oxygen exchange functions of the patient's lungs. The above parameter of lung compliance value refers to the amount of change in lung volume caused by a change in unit pressure.

The comprehensive evaluation value of the patient's current spontaneous breathing ability is a parameter used to characterize the strength of the patient's spontaneous breathing ability. The larger the value, the stronger the patient's spontaneous breathing ability.

The above preset evaluation values are set in advance.

The beneficial effect of the above technical solution is: by monitoring the patient's respiratory wave curve, accurate and effective analysis of the patient's breathing conditions under different concentrations of carbon dioxide is realized, and at the same time, the user can accurately obtain the patient's breathing conditions under different concentrations of carbon dioxide gas and at different time points. Respiratory muscle skill data, to achieve accurate and effective evaluation of the patient's spontaneous breathing ability under different concentrations of carbon dioxide, so as to facilitate timely and effective offline operation of the patient's ventilator according to the evaluation results, improving the safety and effectiveness of offline Sex, but also to ensure the efficiency and effectiveness of offline.

### Embodiment 9:

On the basis of the above-mentioned embodiment 8, this embodiment provides a solution based on mixed gas, a method for taking the machine off-line is to obtain the comprehensive evaluation value of the patient's current spontaneous breathing ability based on the weight value, comprising:
obtaining the comprehensive evaluation value of the patient's current spontaneous breathing ability, and comparing the comprehensive evaluation value of the patient's current spontaneous breathing ability with a preset offline threshold;
wherein if the comprehensive evaluation value of the patient's current spontaneous breathing ability is less than the preset offline threshold, it is determined that the patient does not meet the offline requirements, and at the same time, determine that the patient's current comprehensive evaluation value of spontaneous breathing ability is different from the preset offline threshold a threshold difference, and based on the difference, estimate the duration of use of the ventilator device by the patient;
feeding back the estimated result to the patient's smart terminal;
otherwise, it is determined that the patient meets the off-line requirement, and the off-line operation on the patient is completed.

In this embodiment, the preset threshold is set in advance and is used to measure whether the patient meets the weaning condition.

The beneficial effect of the above technical solution is: by providing a reference basis for whether offline operation can be performed according to the evaluation results, it is convenient to ensure that the offline operation is performed under the premise of patient safety, and the safety and efficiency of solving the patient's ventilator offline are improved.

### Embodiment 10:

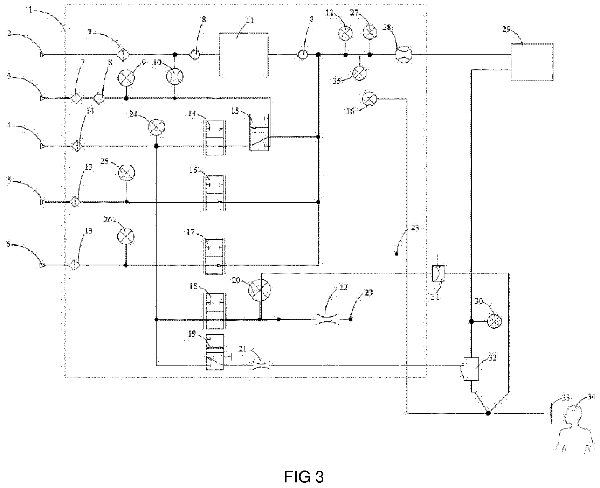

As shown in Fig. 3, the embodiment provides a device for solving ventilator off-line problems on patients based on mixed gases, comprising:
a gas mixing module configured to mix carbon dioxide and oxygen based on the initial mixing concentration and delivering the mixed gas to the patient based on the ventilator device;
a concentration changing module, configured to monitor the patient's partial pressure of carbon dioxide in real time based on the delivery result, and to change the initial mixed concentration of carbon dioxide and oxygen in a gradient based on the partial pressure of carbon dioxide; and
an evaluation module configured to obtain the patient's respiratory muscle function data based on the gradient change result, evaluate the respiratory muscle function data, and complete the patient's ventilator off-line based on the evaluation result.

In this embodiment, a device structure diagram for solving the off-line of a patient ventilator based on mixed gas is shown in Fig. 3: among them, the ventilator system device body 1, air source inlet 2, low-pressure oxygen source 3, high-pressure oxygen gas source 4, first treatment gas port 5, second treatment gas port 6, first filter 7, check valve 8, first pressure sensor 9, first flow sensor 10, blower 11, second pressure sensor 12, The second filter 13, the first flow control valve 14, the direction control valve 15, the second flow control valve 16, the third flow control valve 17, the fourth flow control valve 18, the on-off valve 19, the third pressure sensor 20, First throttle valve 21, second throttle valve 22, pipe outlet 23, second pressure sensor 24, second pressure sensor 25, second pressure sensor 26, oxygen sensor 27, second flow sensor 28, heating humidifier 29 , respiratory pressure sensor 30, exhaust valve 31, drug nebulizer 32, breathing catheter 33, inspiratory pipeline 34, oxygen (or carbon dioxide) concentration sensor 35

The beneficial effect of the above technical solution is: by mixing a certain concentration of carbon dioxide gas and oxygen, the patient's respiratory nerve center can be stimulated, so as to ensure the stimulation of the patient's active breathing, improve the stability of the treatment, and ensure the recovery of the patient's respiratory function. The effect and efficiency of the machine can reduce the pain of patients when they are offline.

## Claims

1. A device for solving ventilator off-line problems on patients based on mixed gases, comprising:
a gas mixing module configured to mix carbon dioxide and oxygen based on an initial mixing concentration and delivering a mixture to a patient based on a ventilator device (1);
a concentration changing module, configured to monitor a patient's partial pressure of carbon dioxide in real time based on the delivery result, and changing a gradient of an initial mixed concentration of carbon dioxide and oxygen based on the partial pressure of the carbon dioxide; and
an evaluation module configured to obtain the patient's respiratory muscle function data and evaluating a respiratory muscle function data based on the gradient change result, the respiratory muscle function data refers to the patient's ability to breathe spontaneously after receiving the mixed gas of carbon dioxide and oxygen at different concentrations and complete the off-line of the patient's ventilator based on an evaluation result that the weaning of the ventilator can be performed when the patient's respiratory muscles can reach a normal standard,
wherein the concentration change module is configured to perform the following steps:
based on the ventilator device (1), continuously inputting the mixed gas of the initial concentration to the patient for a preset period of time, and monitoring the partial pressure of carbon dioxide of the patient in real time, wherein the partial pressure of carbon dioxide comprises an arterial partial pressure of carbon dioxide and end-tidal partial pressure of carbon dioxide;
determining target values of the arterial partial pressure of carbon dioxide and the end-tidal partial pressure of carbon dioxide of the patient at different time points based on the monitoring results, wherein the target value refers to the specific number of partial pressure of carbon dioxide of the patient at different time points, and obtaining a numerical sequence of the partial pressure of carbon dioxide of the patient within a preset time period;
matching a target chart from the preset chart template library based on the numerical sequence, wherein the target chart refers to a chart suitable for displaying the patient's partial pressure of carbon dioxide, and determining the horizontal and vertical coordinate legends of the two-dimensional coordinates of the target chart based on the attribute information of the preset time period and the target value of the numerical sequence, among them, the legend on the abscissa is time, and the legend on the ordinate is the target value of the numerical sequence;
determining the configuration parameters of the target chart, and performing format conversion on the numerical sequence of the partial pressure of carbon dioxide based on the configuration parameters to obtain the partial pressure of carbon dioxide visual graph;
determining a maximum value and a minimum value of the partial pressure of carbon dioxide of the patient in the preset time period based on the partial pressure of carbon dioxide visual graph, and determining fluctuation range of the patient's partial pressure of carbon dioxide based on the maximum value and the minimum value;
comparing the fluctuation range with a standard allowable range;
wherein if the fluctuation range is not within the standard allowable range, it is determined that the inhalation of the mixed gas by the patient is abnormal; and
otherwise, it is determined that there is no abnormality in the patient's inhalation of the mixed gas, and at the same time, the pH value in the patient's body is obtained;
determining the difference between the pH value and the standard pH value, and when the difference is within the preset allowable fluctuation range, determining that the patient has adapted to the mixed gas of the initial mixed concentration, and providing feedback on the determination result to the ventilator device (1); and
based on the received feedback determination result, controlling the ventilator device (1) to perform gradient change on the initial mixed concentration of carbon dioxide and oxygen, and continuously monitor the partial pressure of carbon dioxide of the patient until the initial mixed concentration reaches the maximum allowable value of gradient change, wherein the gradient change refers to sequentially increasing the concentration of carbon dioxide in an allowable range of from 1% to 6%.

## Patentansprüche

1. Vorrichtung zur Lösung von Offline-Problemen des Beatmungsgeräts bei Patienten basierend auf gemischten Gasen, umfassend:
ein Gasmischmodul, das so konfiguriert ist, dass es Kohlendioxid und Sauerstoff basierend auf einer anfänglichen Mischkonzentration mischt und einem Patienten basierend auf einer Beatmungsvorrichtung (1) eine Mischung abgibt;
ein Konzentrationsänderungsmodul, das so konfiguriert ist, dass es den Partialdruck von Kohlendioxid eines Patienten in Echtzeit basierend auf dem Verabreichungsergebnis überwacht und einen Gradienten einer anfänglichen Mischkonzentration von Kohlendioxid und Sauerstoff basierend auf dem Partialdruck des Kohlendioxids ändert; und
ein Auswertungsmodul, das so konfiguriert ist, dass es die Daten zur Atemmuskelfunktion des Patienten erhält und basierend auf dem Gradientenänderungsergebnis die Daten zur Atemmuskelfunktion auswertet, wobei sich die Daten zur Atemmuskelfunktion auf die Fähigkeit des Patienten beziehen, spontan zu atmen, nachdem er das gemischte Gas aus Kohlendioxid und Sauerstoff in verschiedenen Konzentrationen empfangen hat, und den Offline-Betrieb des Beatmungsgeräts des Patienten basierend auf einem Auswertungsergebnis beendet, dass die Entwöhnung vom Beatmungsgerät durchgeführt werden kann, wenn die Atemmuskeln des Patienten einen normalen Standard erreichen können,
wobei das Konzentrationsänderungsmodul so konfiguriert ist, dass es die folgenden Schritte durchführt:
basierend auf der Beatmungsvorrichtung (1), kontinuierliches Zuführen des gemischten Gases der Anfangskonzentration zum Patienten für einen voreingestellten Zeitraum und Überwachen des Kohlendioxid-Partialdrucks des Patienten in Echtzeit, wobei der Kohlendioxid-Partialdruck einen arteriellen Kohlendioxid-Partialdruck und einen endtidalen Kohlendioxid-Partialdruck umfasst;
Bestimmen von Zielwerten des arteriellen Kohlendioxid-Partialdrucks und des endtidalen Kohlendioxid-Partialdrucks des Patienten zu verschiedenen Zeitpunkten basierend auf den Überwachungsergebnissen, wobei sich der Zielwert auf die spezifische Anzahl des Kohlendioxid-Partialdrucks des Patienten zu verschiedenen Zeitpunkten bezieht, und Erhalten einer numerischen Sequenz des Kohlendioxid-Partialdrucks des Patienten innerhalb eines voreingestellten Zeitraums;
Abgleichen eines Zieldiagramms aus der voreingestellten Diagrammvorlagenbibliothek basierend auf der numerischen Sequenz, wobei sich das Zieldiagramm auf ein Diagramm bezieht, das geeignet ist, den Kohlendioxid-Partialdruck des Patienten anzuzeigen, und Bestimmen der horizontalen und vertikalen Koordinatenlegenden der zweidimensionalen Koordinaten des Zieldiagramms basierend auf den Attributinformationen des voreingestellten Zeitraums und des Zielwerts der numerischen Sequenz, darunter ist die Legende auf der Abszisse Zeit, und die Legende auf der Ordinate ist der Zielwert der numerischen Sequenz;
Bestimmen der Konfigurationsparameter des Zieldiagramms und Durchführen einer Formatumwandlung auf der numerischen Sequenz des Kohlendioxid-Partialdrucks basierend auf den Konfigurationsparametern, um das visuelle Kohlendioxid-Partialdruckdiagramm zu erhalten;
Bestimmen eines Maximalwerts und eines Minimalwerts des Kohlendioxid-Partialdrucks des Patienten in dem voreingestellten Zeitraum basierend auf dem Kohlendioxid-Visualgraphen-Partialdruck und Bestimmen eines Schwankungsbereichs des Kohlendioxid-Partialdrucks des Patienten basierend auf dem Maximalwert und dem Minimalwert;
Vergleichen des Schwankungsbereichs mit einem zulässigen Standardbereich;
wobei bestimmt wird, dass die Inhalation des gemischten Gases durch den Patienten abnormal ist, wenn der Schwankungsbereich nicht innerhalb des zulässigen Standardbereichs liegt; und
andernfalls bestimmt wird, dass bei der Inhalation des gemischten Gases durch den Patienten keine Anomalie vorliegt, und gleichzeitig der pH-Wert im Körper des Patienten erhalten wird; Bestimmen der Differenz zwischen dem pH-Wert und dem Standard-pH-Wert, und wenn die Differenz innerhalb des voreingestellten zulässigen Schwankungsbereichs liegt, Bestimmen, dass sich der Patient an das gemischte Gas der anfänglichen Mischkonzentration angepasst hat, und der Beatmungsvorrichtung (1) eine Rückmeldung über das Bestimmungsergebnis gegeben wird; und
basierend auf dem empfangenen Ergebnis der Rückkopplungsbestimmung, das Steuern der Beatmungsvorrichtung (1), um eine Gradientenänderung an der anfänglichen Mischkonzentration von Kohlendioxid und Sauerstoff durchzuführen, und kontinuierliches Überwachen des Kohlendioxid-Partialdrucks des Patienten, bis die anfängliche Mischkonzentration den maximal zulässigen Wert der Gradientenänderung erreicht, wobei sich die Gradientenänderung auf eine sequenzielle Erhöhung der Konzentration von Kohlendioxid in einem zulässigen Bereich von 1 % bis 6 % bezieht.

## Revendications

1. Dispositif de résolution de problèmes de sevrage de ventilateur chez des patients sur la base d'un mélange gazeux, comportant :
un module de mélange de gaz configuré pour mélanger du dioxyde de carbone et de l'oxygène sur la base d'une concentration de mélange initiale et délivrer un mélange à un patient sur la base d'un ventilateur (1) ;
un module de changement de concentration, configuré pour surveiller la pression partielle de dioxyde de carbone d'un patient en temps réel sur la base du résultat d'administration, et modifier un gradient d'une concentration mélangée initiale de dioxyde de carbone et d'oxygène sur la base de la pression partielle du dioxyde de carbone ; et
un module d'évaluation configuré pour obtenir des données de fonction musculaire respiratoire du patient et évaluer des données de fonction musculaire respiratoire sur la base du résultat de changement de gradient, les données de fonction musculaire respiratoire font référence à la capacité du patient à respirer spontanément après avoir reçu le mélange gazeux de dioxyde de carbone et d'oxygène à différentes concentrations et terminer le sevrage de ventilateur du patient sur la base d'un résultat d'évaluation selon lequel le sevrage de ventilateur peut être réalisé lorsque les muscles respiratoires du patient peuvent atteindre une norme normale,
dans lequel le module de changement de concentration est configuré pour réaliser les étapes suivantes :
sur la base du ventilateur (1), l'introduction continue du mélange gazeux de la concentration initiale chez le patient pendant une période prédéfinie, et la surveillance de la pression partielle de dioxyde de carbone du patient en temps réel, dans lequel la pression partielle de dioxyde de carbone comporte une pression partielle artérielle de dioxyde de carbone et une pression partielle en fin d'expiration de dioxyde de carbone ;
la détermination de valeurs cibles de la pression partielle artérielle de dioxyde de carbone et de la pression partielle en fin d'expiration de dioxyde de carbone du patient à différents moments sur la base des résultats de surveillance, dans lequel la valeur cible fait référence au nombre spécifique de pression partielle de dioxyde de carbone du patient à différents moments, et l'obtention d'une séquence numérique de la pression partielle de dioxyde de carbone du patient dans une période prédéfinie ;
la mise en correspondance d'un graphique cible à partir de la bibliothèque de modèles de graphique prédéfinis sur la base de la séquence numérique, dans lequel le graphique cible fait référence à un graphique approprié pour afficher la pression partielle de dioxyde de carbone du patient, et la détermination des légendes de coordonnées horizontales et verticales des coordonnées bidimensionnelles du graphique cible sur la base des informations d'attribut de la période prédéfinie et de la valeur cible de la séquence numérique, parmi lesquelles la légende sur l'abscisse est le temps, et la légende sur l'ordonnée est la valeur cible de la séquence numérique ;
la détermination des paramètres de configuration du graphique cible, et la réalisation d'une conversion de format sur la séquence numérique de la pression partielle de dioxyde de carbone sur la base des paramètres de configuration pour obtenir le graphique visuel de pression partielle de dioxyde de carbone ;
la détermination d'une valeur maximale et d'une valeur minimale de la pression partielle de dioxyde de carbone du patient dans la période prédéfinie sur la base du graphique visuel de pression partielle de dioxyde de carbone, et la détermination d'une plage de fluctuation de la pression partielle de dioxyde de carbone du patient sur la base de la valeur maximale et de la valeur minimale ;
la comparaison de la plage de fluctuation à une plage admissible standard ;
dans lequel si la plage de fluctuation n'est pas dans la plage admissible standard, il est déterminé que l'inhalation du mélange gazeux par le patient est anormale ; et
sinon, il est déterminé qu'il n'y a pas d'anomalie dans l'inhalation du mélange gazeux par le patient, et en même temps, la valeur de pH dans le corps du patient est obtenue ; la détermination de la différence entre la valeur de pH et la valeur de pH standard, et lorsque la différence est dans la plage de fluctuation admissible prédéfinie, la détermination que le patient s'est adapté au mélange gazeux de la concentration mélangée initiale, et la fourniture d'un retour d'informations sur le résultat de détermination au ventilateur (1) ; et
sur la base du résultat de détermination de retour d'informations reçu, la commande du ventilateur (1) pour réaliser un changement de gradient sur la concentration mélangée initiale de dioxyde de carbone et d'oxygène, et la surveillance continue de la pression partielle de dioxyde de carbone du patient jusqu'à ce que la concentration mélangée initiale atteigne la valeur admissible maximale de changement de gradient, dans lequel le changement de gradient fait référence à l'augmentation séquentielle de la concentration de dioxyde de carbone dans une plage admissible de 1 % à 6 %.
